# EUROPEAN PATENT APPLICATION

(11) **EP 2 042 178 A1**
(43) Date of publication of application: **01.04.2009**
(21) Application number: 07291152.2
(22) Date of filing: 26.09.2007
(51) Int. Cl.: A61K 31/513, A61K 45/06, A61P 27/06

(54) **Ocular formulations comprising 5-chlorouracil and use thereof**

(71) Applicant: INSTITUT NATIONAL DE LA SANTE ET DE LA RECHERCHE MEDICALE (INSERM), 75013 Paris (FR); UNIVERSITE DE GENEVE, 1211 Geneve 4 (CH)
(72) Inventor: Behar, Francine, 75016 Paris (FR); Berdugo, Marianne, 75019 Paris (FR); Felt, Olivia, 66420 Le Barcarès (FR); Bossy, Leila, 1204 Geneva (CH); Gurny, Robert, 1204 Geneva (CH)
(74) Representative: reuteler & cie SA

(57) **Abstract**

The present invention is related to new ocular formulations. In particular, the invention provides a use of 5-chlorouracil for the preparation of a pharmaceutical composition for the treatment of glaucoma, especially associated with glaucoma filtering surgery. More particularly, the present invention provides novel pharmaceutical compositions of 5-chlorouracil and use of these in the preparation of pharmaceutical compositions for the treatment of glaucoma, especially associated with glaucoma filtering surgery.

## Description

### Field of the Invention

The present invention relates to new ocular formulations useful for the treatment of glaucoma. In particular, the invention provides a use of 5-chlorouracil (5-CU) for the preparation of a pharmaceutical composition for the treatment of glaucoma, in combination with glaucoma filtering surgery. Further, the present invention provides novel pharmaceutical compositions of 5-chlorouracil and use of these in the preparation of pharmaceutical compositions for the treatment of glaucoma, in combination with glaucoma filtering surgery.

### Background of the Invention

Glaucoma is usually, but not always, associated with elevated pressure in the eye (intra-ocular pressure or IOP). Generally, it is this elevated eye pressure that leads to damage of the eye (optic) nerve. In some cases, glaucoma may occur in the presence of normal eye pressure. This form of glaucoma is believed to be caused by poor regulation of blood flow to the optic nerve. Glaucoma is the leading cause of irreversible blindness. In fact, as many as 6 million individuals are blind in both eyes from this disease. Glaucoma affects 2% of the population aged 40 and over and the percentage of afflicted people rises with age. In the United States alone, about over 3 million people have glaucoma.

Eyedrops to lower pressure in the eye are the mainstay treatment but 10% of patients require surgery to prevent blindness. There are several forms of surgical glaucoma therapies which are used to treat glaucoma, among which glaucoma filtering surgery (GFS) or trabeculectomy which is a delicate microsurgical procedure, is the most common.

Glaucoma filtering surgery consists in the production of a filtration fistula (a small piece of the clogged trabecular meshwork is removed) to facilitate the outflow of the aqueous humor from the interior chamber to reduce intraocular pressure. For the new filtering pathway, a small filtering bleb is created from conjunctival tissue. The filtering bleb is a cyst-like raised area that is placed at the top part of the eye under the upper lid. The new drainage system allows fluid to leave the eye, enter the bleb, and then pass into the capillary blood circulation (thereby lowering the eye pressure). Trabeculectomy is the most commonly performed glaucoma surgery. If successful, it is the most effective means of lowering the eye pressure. However, a wound healing process can seal the surgical site and result in failure of the operation. Fibroblast proliferation plays an important role in this wound healing and scarring process that takes place along the years following glaucoma surgery. Further, leaks in the conjunctival filtering blebs may occur as either an early or late complication of glaucoma filtering surgery. These leaks decrease the ability of the glaucoma filtering surgery to regulate IOP and to control glaucoma symptoms.

Various pharmacological agents such as antimetabolites and anti-inflammatory steroids have been used to inhibit the growth of fibroblasts *in vitro*. The use of antimitotic agents as an adjunct treatment to glaucoma filtering surgery has become accepted in practice. Many molecules have been studied for their antiproliferative effect on Tenon's fibroblasts, such as 5-fluorouracil (5-FU), mitomycin, 5-fluorouridine, fluoroorotate, sodium butyrate, octreotide, cytosine arabinoside, methotrexate, suramin and doxorubicin. Amongst these, 5-FU and mitomycin C (Wong et al., 1991, Ocul. Pharmacol., 7, 27-39*;* Saika et al., 1997,. Ophthalmic Res., 29, 91-102) are the most frequently used in clinical practice. Topical application of 5-fluorouracil and mitomycin C at the time of surgery has resulted in significant reduction of filtering surgery failures in high-risks patients, but is associated to local adverse effects (Singh et al., 2000, Ophthalmology, 107, 2305-9). Mitomycin C is known to be associated with an increased incidence of postoperative delayed hypotony, ciliary body toxicity, late postoperative endophthalmitis, and endothelial toxicity. Intra-operative 5-FU as an adjunct treatment to trabeculectomy improves the outcome of filtering surgery by reducing fibroblastic activity in Tenon's Capsule. Its first efficacy was shown in randomized controlled trials using of postoperative subconjunctival 5-FU injections.
5-FU is a synthetic pyrimidine analogue, which inhibits the thymidylate synthetase that converts ribonucleotides to desoxyribonucleotides, thus inhibiting DNA synthesis. Since it acts selectively on the growth phase of the cell cycle, only cells in the synthesis phase are affected. The remaining cells can continue to proliferate after exposure to 5-FU. Therefore, repeated applications are often required following per-operative tamponade. However, several adverse effects are observed with 5-FU use. Such adverse effects include corneal epithelial toxicity, ciliary body toxicity, leaking blebs, endophthalmitis, scleral thinning, discomfort and transient cataract (Libre et al., 2003, Am. J. Ophthalmol., 135, 101-2). A sustained release formulation based on a poly(ortho ester) (POE) polymer for combined ocular delivery of dexamethasone sodium phosphate and 5-FU has been studied in rabbits (Zignani et al., 2000, Eur. J. Pharmaceutics and Biopharmaceutics, 50, 251-255).

Glaucoma filtering surgery is the last chance treatment for many late stage patients suffering from glaucoma and the success of this surgical treatment depends on the efficacy of post-surgery treatments. Current medications for post-surgery glaucoma treatment in glaucoma patients are limited and present severe side effects.

Due to the severity of the consequences of a failure of glaucoma filtering surgery -which may lead to partial or complete blindness-, it would be highly desirable to develop new methods and formulations for the post-operative treatment of glaucoma filtering surgery with limited side effects.

### Summary of the Invention

The present invention is directed towards new ocular biocompatible formulations. In particular, the invention provides methods, use and compositions for improving the success of glaucoma filtration surgery, e.g. for preventing or partially preventing the side effects or post-operative symptoms of glaucoma filtering surgery. More particularly, the present invention provides a use of 5-chlorouracil and novel pharmaceutical compositions thereof in the preparation of pharmaceutical compositions for the treatment of glaucoma, especially per-operative treatment of glaucoma filtering surgery.

A first aspect of the invention provides a use of 5-chlorouracil for the preparation of a pharmaceutical composition for the treatment of glaucoma, in combination with glaucoma filtering surgery.

A second aspect of the invention relates to a pharmaceutical composition comprising a therapeutically effective amount of 5-chlorouracil, poly(ortho esters) and a pharmaceutically acceptable carrier or excipient.
A third aspect of the invention resides in a method for treating glaucoma in a subject, comprising the administration of a therapeutically effective amount of 5-chlorouracil or of a pharmaceutical formulation thereof in a subject in need thereof.
A fourth aspect of the invention is related to a method of inhibiting, preventing, and/or treating of conjunctival bleb leaks in a glaucoma subject following filtering surgery, comprising the administration of a therapeutically effective amount of 5-chlorouracil or of a pharmaceutical formulation thereof in a subject in need thereof.
A fifth aspect of the invention is related to a method of inhibiting, preventing, and/or treating optical nerve damage in a glaucoma subject, comprising the administration of a therapeutically effective amount of 5-chlorouracil or of a pharmaceutical formulation thereof in a subject in need thereof.
A sixth aspect of the invention provides a method of inhibiting, preventing, and/or treating the production of excessive ocular fibrous or scar tissue in a glaucoma subject following filtering surgery, comprising the administration of a therapeutically effective amount of 5-chlorouracil or of a pharmaceutical formulation thereof in a subject in need thereof.
A seventh aspect of the invention provides a method of inhibiting, preventing, and/or treating the elevation of IOP in a glaucoma subject, comprising administering to the subject a therapeutically effective amount of 5-chlorouracil or of a pharmaceutical formulation thereof.
An eighth aspect of the invention provides a method of treating or alleviating the symptoms of glaucoma using an implant according to the invention.
A ninth aspect of the invention provides an administration kit comprising (a) a container containing 5-chlorouracil and a formulation thereof according to the invention; and (b) a container containing a pharmaceutically acceptable medium solution.
A tenth aspect of the invention provides a biodegradable implant for placement in an eye coated with a pharmaceutical formulation of 5-chlorouracil.

A eleventh aspect of the invention provides an implant kit comprising: (a) an implant according to the invention; and (b) a delivery apparatus for the said implant.
An eleventh aspect of the invention provides an injection kit according to the invention.

### Description of the figures

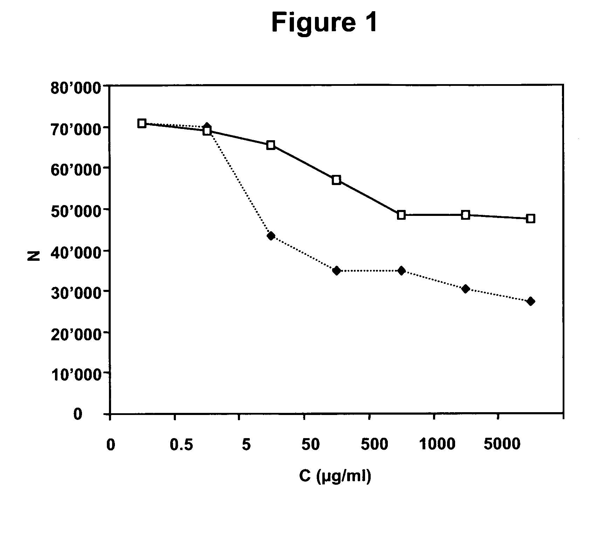

**Figure 1** represents the number of living cells (N) as analyzed by MTT test according to Example 2 after a 24 hour-incubation of various concentrations (C) (in µg/ml) of 5-CU (empty squares) or 5- FU (black lozenges).

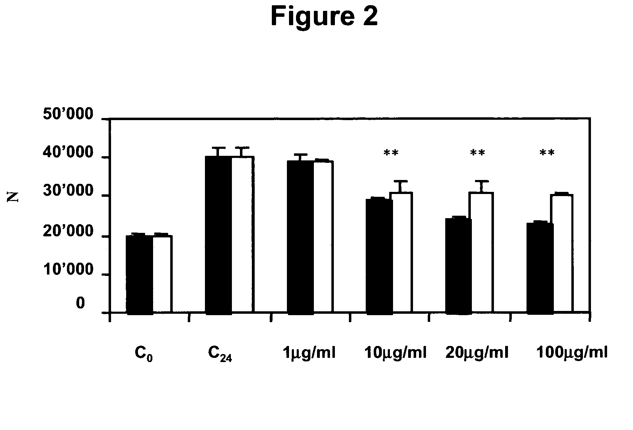

**Figure 2** represents the number of cells (N) from *ex-vivo* taken rabbit Tenon's fibroblasts after a 24 hour-incubation as described in Example 2, with various concentrations of 5-FU (dark histograms) or 5-CU (white histograms). X legend: C₀ and C₂₄ indicate control samples at 0 h and 24 h, respectively and the concentrations indicate for which the number of cells was counted.

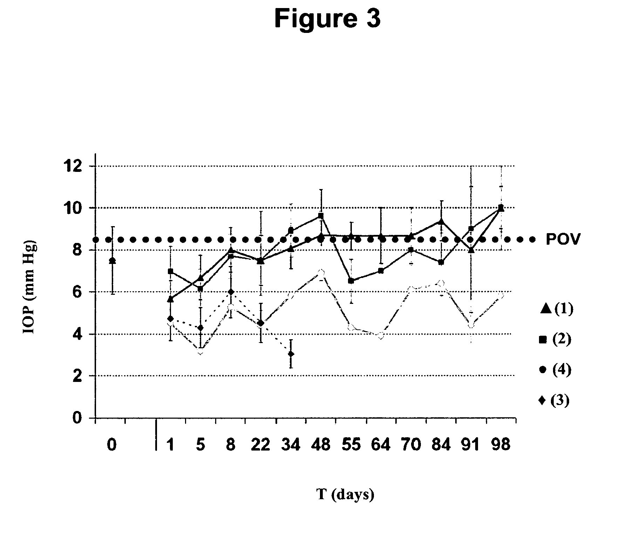

**Figure 3** represents the follow up of the mean intraocular pressure (IOP in mm Hg) versus time (T) in days before trabeculectomy (day 0) and within a 5 month post-operative follow-up after filtering surgery on rabbits as described in Example 4, using Goldmann aplanation tonometry. Group 1 (1) = trabeculectomy alone; Group 2 (2) = trabeculectomy + POE IV; Group 3 (3) = trabeculectomy + POE IV/1% 5-FU; Group 4 (4) = trabeculectomy + POE IV/1% 5-CU. "POV" line = pre-operative mean IOP. Results are expressed as means +/sem.

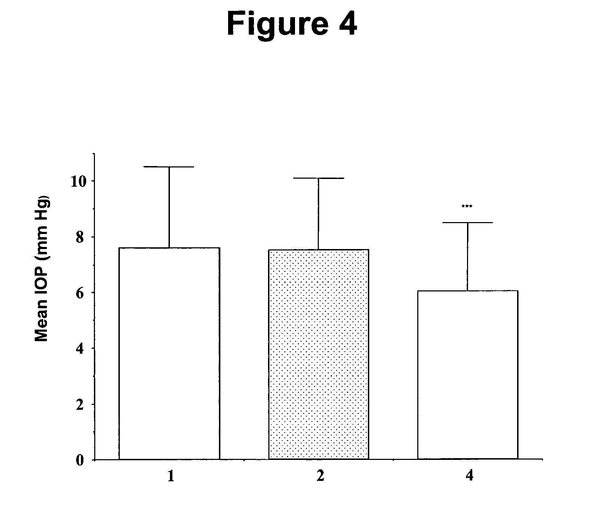

**Figure 4** represents the mean of intraocular pressures (IOP in mm Hg) during the long-term follow-up study as described in Example 4 (150 days after filtering surgery). Controls (trabeculectomy alone: (1) grey histogram; trabeculectomy with injection of POE IV alone: (2) dotted histogram, as compared to the group which had received the POE IV + 5-CU composition of the invention: (4) white histogram (P<0.0001).

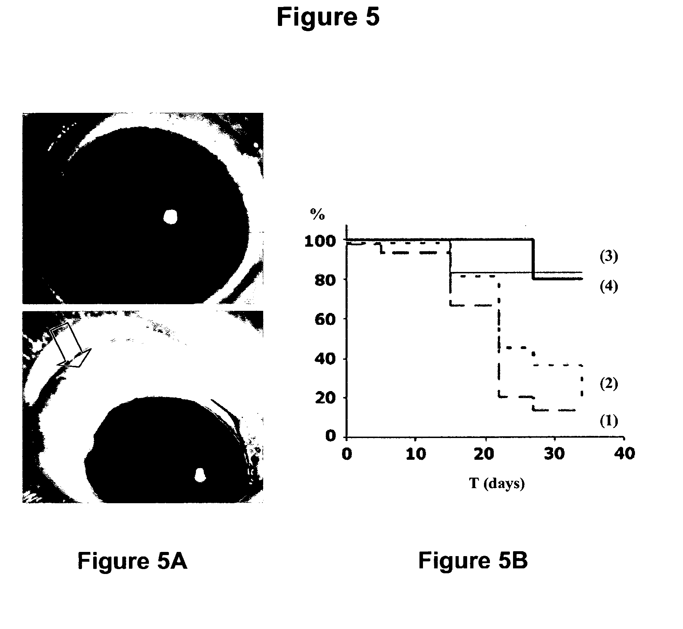

**Figure 5** **represents** slit lamp photographs of the rabbit eyes 4 weeks after filtering surgery (Figure 5A) and bleb survival curves expressed in percentage of eyes (%) with a persistant bleb versus time (T) in days after trabeculectomy (Figure 5B) as described in Example 4.
**Figure 5A****:** Group 1 (top) = trabeculectomy alone; Group 4 (bottom) = trabeculectomy + POE IV/1% 5-CU where a well-shaped bleb is represented by an arrow. **Figure 5B****:** Group 1 (1) = trabeculectomy alone; Group 2 (2) = trabeculectomy + POE IV; Group 3 (3) = trabeculectomy + POE IV/1% 5-FU; Group 4 (4) = trabeculectomy + POE IV/1% 5-CU.

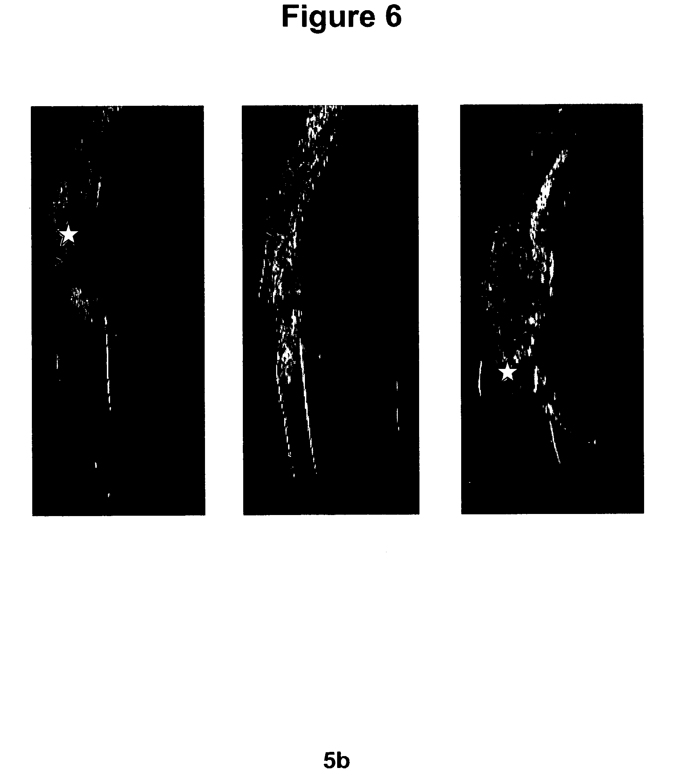

**Figure 6** shows high-resolution ultrasonography images (80 MHz nominal frequency, 7.5-mm focal length, 3-mm aperture) of *ex-vivo* obtained eyeballs immersed in saline (0.9 % NaCl), and positioned on an adjustable holder as described in Example 4. The trabeculectomy site is facing the transducer. **A:** Acutely after trabeculectomy alone (group 1); **B**: 11 months after trabeculectomy alone; **C:** 11 months after trabeculectomy + POE IV/1% 5-CU. Sagittal sections of the eyes: thin arrow = sclera; thick arrow = cornea; arrowhead = iris. White stars indicate the site of fresh trabeculectomy (in A), that is not seen anymore in B, but can still be observed in C.

### Detailed Description of the invention

The term "glaucoma filtering surgery" comprises surgery treatment of glaucoma by trabeculectomy or sclerectomy or other penetrating or non-penetrating filtering surgery.
As used herein, "treatment" and "treating" and the like generally mean obtaining a desired pharmacological and physiological effect. The effect may be prophylactic in terms of preventing or partially preventing the side effects or post-operative symptoms of glaucoma filtering surgery such as leakage of a conjunctive bleb or the excessive formation of ocular fibrous or scar tissue leading to an increase in IOP, and/or may be therapeutic in terms of a partial or complete cure of glaucoma or post-operative symptoms thereof. The term "treatment" as used herein covers any treatment of a disease in a subject, particularly a human, and includes: (a) preventing the post-operative symptoms of glaucoma filtering surgery from occurring in a subject which may be predisposed but has not yet been diagnosed as having them; (b) inhibiting the post-operative symptoms of glaucoma filtering surgery, i.e., preventing or arresting their development; or relieving from those post-operative symptoms, i.e., causing regression of thereof. The term "treatment" as used herein further covers the treatment of glaucoma, especially in combination with glaucoma filtering surgery.

The term "subject" as used herein refers to mammals. For examples, mammals contemplated by the present invention include human, primates, domesticated animals such as cattle, sheep, pigs, horses and the like.
The term "glaucoma subject" as used herein refers to a subject suffering from glaucoma and which need to undergo or has undergone filtering surgery.
The term "efficacy" of a treatment or a use according to the invention can be assessed for example based on persistence of a low post-operative IOP in response to a use or a method according to the invention. For example, the efficacy of a method or a use according to the invention can be assessed by a post-operative IOP remaining lower than the pre-operative value, and within the range of normal IOP, normal IOP being defined for each animal species or for humans. Typically, normal IOP for humans is in the range of 10 to 19 mmHg pre-operative IOP values are in the range of about 22-40 mmHg.
The term "implant" as used herein comprises an implant for placement in an eye suitable for post-operative treatment of glaucoma filtering surgery. For example, an implant in the context of the invention is an ocular or ophthalmic implant such as an ocular aqueous humor drainage device such as described in Bourges et al., 2006, Adv. Drug Deliv. Rev., 58(11):1182-202.

### 5-Chlorouracil

5-Chlorouracil or 5-chloro-1H-pyrimidine-2,4-dione has been disclosed as an enzyme inhibitor and used an antiviral and antitumour agent.

### Poly(ortho esters)

Poly(ortho esters) or POEs are a family of hydrophobic, biocompatible and bioerodible polymers used as a sustained delivery system (Merkli et al., 1993, J. Biomater. Sci. Polym. Ed., 4:505-516*;* Ng et al., 1997, Macromolecules, 30: 770-772).
Typically, POEs suitable for use in a formulation according to the invention are such as those described in Einmahl et al., 2001, Adv. Drug Deliv. Rev., 53(1):45-73 and in Einmahl et al., 2001, Invest. Ophthalmol. Vis. Sci., 695-700*.*
For example, POEs used in a formulation according to the invention are selected from "first", "second", "third" and "fourth" generation POEs (respectively named POE I, POE II, POE III and POE IV), such as those described in Heller et al., 2002, Advanced Drug Delivery Reviews 54 (2002) 1015-1039*.* In a particular aspect of the invention, the POEs used in a formulation according to the invention are selected from POEs III and POEs IV. Typically suitable POEs used in a formulation according to the invention are a molecular weight autocatalyzed POEₓLA_{y} wherein x ranges from about 70 to about 90 and y ranges from about 30 to about 10, the molecular weight of those POEs ranging from about 3'000 to about 50'000 g/mol, typically from about 3'000 to about 10'000 g/mol.
Semi-solid POEs or solid POEs mixed with an adequate pharmaceutically acceptable excipient(s) are suitable POEs in the context of the invention.

### Compositions

The invention provides 5-chlorouracil pharmaceutical formulations, use of 5-chlorouracil, pharmaceutical formulations thereof and methods for treating a glaucoma subject, preferably a mammalian patient, and most preferably a human patient who is in need to undergo or has undergone glaucoma filtering surgery. Uses, methods and formulations according to the invention are especially useful to treat or prevent an increase of IOP following that surgery and leakage of a conjunctival bleb in the eye of said subject.
Pharmaceutical compositions of the invention contain poly(ortho esters) in any form described herein. Compositions of this invention may further comprise one or more pharmaceutically acceptable additional ingredient(s) such as stabilizers, antimicrobial agents, buffers, coloring agents, flavoring agents, adjuvants, and the like.
The pharmaceutical composition of the invention, together with a conventionally employed adjuvant, carrier, diluent or excipient may be placed into the form of pharmaceutical compositions and unit dosages thereof, and in such form may be employed as liquids such as solutions, suspensions, emulsions, or in the form of sterile injectable solutions for ocular use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. Compositions according to the invention are preferably injectable, notably suitable for *intra* or *peri* ocular injections, and especially suitable for subconjunctival injections at the site of glaucoma filtering surgery.
Compositions of this invention may also be liquid formulations including, but not limited to, aqueous or oily suspensions, solutions and emulsions. Liquid forms suitable for injection may include a suitable aqueous or non-aqueous vehicle with buffers, suspending and dispensing agents, colorants, and the like. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agent include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Non-aqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in Part 5 of Remington's Pharmaceutical Sciences, 20th Edition, 2000, Marck Publishing Company, Easton, Pennsylvania, which is incorporated herein by reference.
Injectable compositions are typically based upon injectable sterile saline or phosphate-buffered saline or other injectable carriers known in the art.
Compositions of this invention may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. Compositions of this invention may also be formulated as a depot preparation, which may be administered by implantation or by injection.
The compounds of this invention can also be administered in sustained release forms or from sustained release drug delivery systems. A description of representative sustained release materials can also be found in the incorporated materials in *Remington's Pharmaceutical Sciences.*

Solid POEs can be used as a mixture with an adequate pharmaceutically acceptable excipient such as sesame oil.

### Mode of administration

Compositions of this invention may be administered by injection in the eye undergoing glaucoma filtering surgery, before, during or after surgery. The compositions of this invention may also be administered in the form of an ocular implant, which allows slow release of the compositions as well as a slow controlled i.v. infusion. In a preferred embodiment, a composition according to the invention is administered by peri-ocular or intra-ocular injection. Ocular injection sites may be sub-conjonctival, intra-cameral, intra-vitreal, or supra-choroidal, preferably sub-conjunctival.
Compositions according to the invention may be also administered on a collagen sponge or a cellulose sponge such as described in Lachkar et al., 1997, Br. J. Ophthalmol. 81:555-558 and in Mora et al., 1996, Ophthalmology, 103:963-70. Typically, a sponge is soaked into a 5-chlorouracil and pharmaceutical formulation thereof solution and then placed between the conjunctiva and the sclera over the filtration site (or planned filtration site) for about 5 minutes.
This invention is further illustrated by the following examples that are not intended to limit the scope of the invention in any way.
The dosage administered, as single or multiple doses, to an individual will vary depending upon a variety of factors, including pharmacokinetic properties, patient conditions and characteristics (sex, body weight, health, size), extent of symptoms, concurrent treatments, previous medical or surgical treatments, frequency of treatment and the effect desired.

### Combination

According to the invention, 5-chlorouracil and pharmaceutical formulation thereof can be administered alone or in combination with a co-agent useful in the treatment of glaucoma and/or post-operative glaucoma filtering surgery, such as substances useful in the treatment and/or prevention of elevation of IOP or leakage of a conjunctival bleb in the eye e.g. for example a co-agent selected from anti mitotic agents, antibodies or antibodies fragments (blocking TNFα, TGFβ, INF, Interleukin 1, 6, VEGF, P1GF or any other cytokines, or growth factors such as FGF), soluble receptors against TNF, TGF, VEGF or any other soluble pro-angiogenic cytokine, agents blocking integrins such as peptides or peptidomimetics, any peptides or peptidomimetics blocking the activity of growth factors, and any poison blocking mitosis such as saporin.
The invention encompasses the administration of 5-chlorouracil or of a pharmaceutical formulation thereof, wherein 5-chlorouracil or the pharmaceutical formulation thereof is administered to an individual prior to, simultaneously or sequentially with other therapeutic regimens or co-agents useful in the treatment of post-glaucoma surgery (e.g. multiple drug regimens), in a therapeutically effective amount. 5-chlorouracil or the pharmaceutical formulation thereof that are administered simultaneously with said co-agents can be administered in the same or different composition(s) and by the same or different route(s) of administration.

### Patients

In an embodiment, subjects according to the invention are subjects suffering from glaucoma and which need to undergo or have undergone filtering surgery.
In a further embodiment, subjects according to the invention are subjects suffering from excessive ocular fibrous or scar tissue or elevated IOP and which have undergone filtering surgery.
In another further embodiment, subjects according to the invention are subjects suffering from leakage of an ocular conjunctival bleb and which have undergone filtering surgery.

### Use according to the invention

Typically, the efficacy of the formulations, uses and methods according to the invention may be assayed in various assays such as through the monitoring of post-operative IOP, in response to a use or a method according to the invention. Such assays have been described in Einmahl et al., 2001, Invest. Ophthalmol. Vis. Sci., 695-700.

In one embodiment, the invention provides a use of 5-chlorouracil for the preparation of a pharmaceutical composition for the treatment of glaucoma, in combination with glaucoma filtering surgery.

In a further embodiment, the invention provides a use according to the invention wherein the composition is a formulation suitable for ocular injection, such as *peri*-ocular or *intra-*ocular injection.

In another further embodiment, the invention provides a use according to the invention
wherein the composition is an ocular formulation suitable for sub-conjunctival ocular injection.

In another further embodiment, the invention provides a use according to the invention
wherein the composition is an ocular formulation suitable for ocular administration with a collagen sponge.

In a further embodiment, the invention provides a use according to the invention wherein the composition is a pharmaceutical formulation of 5-chlorouracil according to the invention.

In another embodiment, the invention provides a pharmaceutical composition comprising a therapeutically effective amount of 5-chlorouracil, poly(ortho esters) and a pharmaceutically acceptable carrier or excipient.

In a further embodiment, the invention provides a pharmaceutical composition according to the invention wherein the poly(ortho esters) are selected from POEs III and POEs IV.

In another further embodiment, the invention provides a pharmaceutical composition according to the invention wherein the poly(ortho esters) is a low molecular weight autocatalyzed POEₓLA_{y} wherein x ranges from about 70 to about 90 (x is typically selected from 70, 80 and 90) and y ranges from about 30 to about 10 (y is typically selected from 10, 20 and 30), the molecular weight of those POEs ranging from about 3'000 to about 50'000 g/mol, typically from about 3'000 to about 10'000 g/mol.

In another further embodiment, the invention provides a pharmaceutical composition according to the invention wherein the pharmaceutical composition comprises a weight amount of 5-chlorouracil selected from about 1, 2, 3, 4, 5, 6, 7, 8, 9 and 10% (wt/wt).

Typically, the pharmaceutical composition comprises a weight amount of 5-chlorouracil of about 1% in weight.

In another further embodiment, the invention provides a pharmaceutical composition according to the invention wherein the pharmaceutical composition comprises a weight amount of poly(ortho esters) or a combination of poly(ortho esters) with an adequate pharmaceutically accepted excipient selected from about 99, 98, 97, 96, 95, 94, 93, 92, 91 and 90% (wt/wt).

In another further embodiment, the invention provides a pharmaceutical composition according to the invention wherein the composition is a formulation suitable for ocular injection, such as sub-conjunctival ocular injection.

In another embodiment, the invention provides a method for treating glaucoma comprising administering of a therapeutically effective amount of 5-chlorouracil or of a pharmaceutical formulation thereof, in a subject in need thereof.

In another embodiment, the invention provides a method of treating and/or preventing leakage of a conjunctival bleb in the eye of a glaucoma subject following filtering surgery, comprising administering of a therapeutically effective amount of 5-chlorouracil or of a pharmaceutical formulation thereof, in a subject in need thereof.

In another embodiment, the invention provides a method of treating and/or preventing optic nerve damage in a glaucoma subject, comprising administering of a therapeutically effective amount of 5-Chlorouracil or of a pharmaceutical formulation thereof, in a subject in need thereof.

In another embodiment, the invention provides a method of inhibiting and/or preventing the production of excessive ocular fibrous or scar tissue in a glaucoma subject following filtering surgery, comprising administering a therapeutically effective amount of 5-chlorouracil or of a pharmaceutical formulation thereof, in a subject in need thereof.

In another embodiment, the invention provides a method of inhibiting and/or inhibiting the elevation of IOP in a glaucoma subject, comprising administering a therapeutically effective amount of 5-chlorouracil or of a pharmaceutical formulation thereof, in a subject in need thereof.

In a further embodiment, the invention provides a method according to the invention which comprises the steps of:
(a) preparing a dosage comprising a therapeutically effective amount of 5-chlorouracil or of a pharmaceutical formulation thereof
(b) administering the dosage to the said subject in need thereof.

In another further embodiment, the invention provides a method according to the invention wherein the therapeutically effective amount of 5-chlorouracil or of a pharmaceutical formulation thereof is administered to the subject, before, during or after filtering surgery.

In another further embodiment, the invention provides a method according to the invention wherein the effective amount of 5-chlorouracil or of a pharmaceutical formulation thereof is administered by *peri*-ocular or *intra*-ocular injection.

In another further embodiment, the invention provides a method according to the invention wherein the effective amount of 5-chlorouracil or of a pharmaceutical formulation thereof is administered by sub-conjunctival ocular injection.

In another further embodiment, the invention provides a method according to the invention wherein the effective amount of 5-chlorouracil or of a pharmaceutical formulation thereof is administered by collagen sponge tamponnade.

In another embodiment, the invention provides a method according to the invention which comprises the administration of a pharmaceutical formulation of 5-chlorouracil according to the invention.

In another embodiment, the invention provides a biodegradable implant for placement in an eye, wherein the implant is coated with a therapeutically effective amount of 5-chlorouracil.

In another embodiment, the invention provides a biodegradable implant according to the invention wherein the implant is coated with a pharmaceutical formulation of 5-chlorouracil.

In another embodiment, the invention provides a biodegradable implant according to the invention wherein the implant is coated with a pharmaceutical formulation of 5-chlorouracil according to the invention.

In another embodiment, the invention provides a biodegradable implant according to the invention, comprising: 5-chlorouracil and poly(ortho esters) polymer. Typically, the biodegradable implant according to the invention wherein 5-chlorouracil makes up between about 1% percent by weight of the biodegradable implant. Typically, such a biodegradable implant according to the invention releases about 30% wt/wt of the 5-chlorouracil within about 30 to 60 days, in an *in vitro* assay such as described in *Zignani et al., 2000, above*.

In another embodiment, the invention provides a method of treating or alleviating the symptoms of glaucoma using an implant according to the invention.

In a further embodiment, the invention provides a method of treating or alleviating the symptoms of glaucoma using an implant according to the invention by:
(a) surgical inserting an implant according to the invention between the sclera and Tenon's tissue of the eye; and
(b) inserting a drainage tube through the surface of the eye and into either the anterior chamber or posterior chamber of the eye to allow fluid to drain from the anterior chamber or posterior chamber into the drainage region of the implant.

In another embodiment, the invention provides an implant kit comprising: (a) an implant according to the invention; and (b) a delivery apparatus for the said implant. Typically, delivery apparatus for an implant according to the invention are as described in Sarodia et al., 2007, Curr. Opin. Ophthalmol. 18(2):152-8*;* Ayyala et al., 2006, Expert Rev. Med. Devices., 3(4):509-21*;* Schwartz et al., 2006, Curr. Opin. Ophthalmol. 17(2):181-9*.*

In another embodiment, the invention provides an administration kit comprising:
(a) a container such as selected from a syringe, a vial or any type of container, containing 5-chlorouracil and a formulation thereof according to the invention; and (b) a container such as selected from a vial, a syringe, a sponge and a polymer matrix containing a pharmaceutically acceptable medium solution. The pharmaceutically acceptable medium solution is typically a solution containing commonly used excipients for injectables such as for examples stabilizers, antimicrobial agents, buffers, coloring agents, flavoring agents, adjuvants, and the like. Typically, in the case where the 5-chlorouracil or the formulation thereof according to the invention is contained in the syringe, the vial contains an injectable medium solution and upon operation of the syringe to administer 5-chlorouracil to a subject, the injectable medium solution in the vial is aspirated into the syringe and mixes with the 5-chlorouracil or the formulation thereof according to the invention before administration into a subject. Alternatively, in the case where an injectable medium solution is contained in the syringe, the vial contains the 5-chlorouracil or the formulation thereof according to the invention and upon operation of the syringe, the injectable medium solution in the syringe is introduced into to the vial and mixes with the 5-chlorouracil or the formulation thereof according to the invention and then, the mixture so obtained is aspirated back into the syringe before administration into a subject. Alternatively, an administration kit according to the invention contains 5-chlorouracil or a formulation thereof according to the invention ready for use.

In a further embodiment, the invention provides an injection kit according, wherein 5-chlorouracil is provided in a form selected from a powder, a lyophilized or compressed pellet, polymer, dispersed forms with or without polymeric biodegradable or non biodegradable and a solution.

In a further embodiment, the invention provides an injection kit according to the invention wherein the kit further comprises a filter for the syringe.

In a further embodiment, the invention provides an injection kit according to the invention wherein the vial further comprises at least one component selected from the group consisting of commonly used pharmaceutically acceptable excipients for injectables.

In a further embodiment, the invention provides an injection kit or an implant kit according to the invention, wherein the kit further comprising instructions for utilization of the kit.

5-CU shows unexpected an antiproliferative effect on fibroblasts, associated to a lower cellular toxicity than the known compounds used for the treatment of post-operative glaucoma surgery symptoms, particularly a lower corneal and conjunctival toxicity. Therefore, 5-CU is very beneficial as an adjunct treatment of glaucoma filtering surgery, in particular when formulated in a slow-release system adapted for ocular use.

Therefore, uses, methods and compositions according to the invention are particularly useful in the treatment of glaucoma, especially in combination with glaucoma filtering surgery.

In another aspect of the invention, the uses, methods and compositions according to the invention are particularly useful in the prevention and/or treatment of scar formation in the ocular filtration bleb and/or drainage fistula following glaucoma surgery.

In another aspect of the invention, the uses, methods and compositions according to the invention are particularly useful in the prevention and/or treatment of elevation of IOP or leakage of a conjunctival bleb in a subject undergoing glaucoma filtering surgery.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.
The invention having been described, the following examples are presented by way of illustration, and not limitation.

### Examples

The following abbreviations refer respectively to the definitions below:
**g** (gram), **mmol** (millimol), **mg** (milligram), µ**g** (microgram), **mHz** (mega Hertz), **ml** (milliliter), **mm** (millimeter), µ**m** (micrometer), **nm** (nanometer), **Rad** (Radiation Absorbance Dose), **wt** (weight), **DETOSU** (3,9-diethylidene-2,4,8,10-tetraoxaspiro[5,5]undecane), **DMEM** (Dulbecco's minimum essential medium), **FBS** (fetal bovine serum), **5-CU** (5-chlorouracil), **5-FU** (5-fluorouracil), FGF (fibroblast growth factor), **GFS** (Glaucoma filtering surgery), **IOP** (Intraocular pressure), **LA** (lactic acid), **MTT** (3-(4,5-dimethylthiazol-2-yl)-2,5-diphenyl tetrazolium bromide), **PBS** (Phosphate Buffer Sulfate), **P1GF** (placenta growth factor), **POE** (poly(ortho esters)), **SEC** (size exclusion chromatography), **TEA** (triethylamine), **THF** (tetrahydrofuran), **TNF** (tumor necrosis factor), **VEGF** (vascular endothelial growth factor).

### Example 1: Cell death mechanism and antimitotic effect induced by 5-CU

The mechanism of action of 5-CU and its antimitotic activity have been compared to a molecule known to have antiproliferative effects on Tenon's fibroblasts: 5-FU.

### Hoechst test and Neutral Red test:

A human conjunctival cell line (*Wong-Kilbourne derivative of Chang conjunctiva;* American Type Culture Collection, Manassas, VA) was cultured under standard conditions (humidified atmosphere of 5% CO₂ at 37°C) in DMEM (Eurobio, Les Ulis, France) supplemented with 10% FBS (Dominique Dutscher, Brumath, France), 0.5% penicillin/streptomycin, and 1% L-Glutamin (Eurobio). Normal culture development was assessed daily by phase-contrast microscopy. Confluent cultures were removed by gentle trypsin incubation, and cells were counted. For cytotoxicity experiments, cells were seeded into 96-well culture plates (18'000 cells per well; Nunc, Roskild, Denmark) until sub-confluence (culture surface covering nearly 70%) and were then exposed to the different formulations.
Experiments were performed by microplate cold light fluorometry (Fluorolite 1000 Dynex; Issy-les-Moulineaux, France), as previously validated (Debbasch et al., 2001, Invest. Ophthalmol. Vis. Sci., 42, 642-652). According to the recommendations of the European Center for the Validation of Alternative Methods (ECVAM) and to previously validated methods in the Chang cell line, two cellular markers were evaluated: cellular viability and chromatin condensation. Cellular viability was evaluated with a neutral red (Fluka, Ronkonkoma, NY) test with fluorometric detection (excitation: 535 nm; emission: 600 nm). Hoechst 33342 (Molecular Probes, Eugene, OR) is an intercalating dye, which allows the determination of the total chromatin quantity variations and the degree of chromatin condensation. Neutral red was used on cells at a final concentration of 10 µg/ml (excitation: 360 nm; emission: 450 nm). 0.5 mg/ml propidium iodide (Roche Diagnostics Corp., Meylan, France) was added to the Hoechst solution, to control necrotic cells, as previously validated.
The supravital uptake of Hoechst 33342 combined with extrusion of propidium iodide was used as an assay for apoptosis as described in Belloc et al., 1994, Cytometry, 17, 59-65. Apoptotic cell death is a mechanism where the intracellular content of the dying cell is kept sequestered. It is defined by a series of cellular changes: (a) the cell shrinks, looses contact with neighbouring cells or surrounding matrix and starts to display intracellular proteins on its surface; (b) the chromatin in the nucleus condenses and the DNA is cleaved into small fragments; (c) the plasma membrane then begins to show a bubbled appearance and small membrane bound bodies break off containing intracellular material which can include nuclear matter and cellular organelles. These fragments are known as "apoptotic bodies" which are quickly removed by phagocytes or by neighbouring cells. Necrotic cell death is an uncontrolled cell death, characterised by: (a) cell swelling and mitochondrial damage leading to rapid depletion of energy levels; (b) a breakdown of homeostatic control; (c) cell membrane lysis and release of the intracellular contents, leading to an inflammatory response, with oedema and damage to the surrounding cells.

In these experiments, cell viability and chromatin condensation were evaluated after a 24 and 72 hour-incubation, with 0, 0.1, 1, 10, 100, and 1000 mg/ml of 5-chlorouracil as compared with the same concentrations of 5-fluorouracil. The value of IC₅₀ (concentration inhibiting 50% of cell population) was used to compare 5-FU and 5-CU cytotoxicities. Dulbecco's minimum essential medium was used as a control, and was used to dilute the tested molecules.

### Chromatin condensation evaluation:

After 24 hours and up to 1 µg/ml of 5-FU or 5-CU, no significant difference in chromatin condensation is observed in human conjunctival cells compared to control cells. Increasing 5-CU concentrations (up to 1'000 µg/ml) induce a very significant increase in Hoechst fluorescence when compared with identical 5-FU concentrations (P=0.0079), indicating that 5-CU induces apoptotic cell death, whereas 5-FU induces necrotic cell death. After 72 hours of exposure, similar chromatin condensation pattern is observed in cells treated with 5-CU at 1 µg/ml to 100 µg/ml. Interestingly, increasing 5-FU concentrations (>1 µg/ml), induced a dose-dependant significant decrease in chromatin condensation after 24 (P=0.0079) or 72 hours (P=0.02) of incubation (P<0.05 considered significant). This test demonstrates that apoptotic death is induced by high 5-CU concentrations (up to 0.1mg/ml) but not by high concentrations of 5-FU. The advantage of an apoptotic cell death is that it may not induce local inflammatory reaction.

### Neutral red test analysis:

In order to compare and quantify 5-CU and 5-FU cytotoxicities, cellular viability was determined. After a 24 hour-incubation and for concentrations up to 10 µg/ml, 5-FU and 5-CU induced similar low cell viability decrease (about 20%). At higher concentrations (100 µg/ml), 5-FU induced 55% of cell death, whilst even a higher 5-CU concentration (1'000 µg/ml) induced only 35% of cell death. This difference is statistically very significant (P=0.0079). In the case of a 72 hour-incubation with 5-FU, cellular viability was significantly decreased at 1 µg/ml and continued to decrease with higher 5-FU concentrations. Contrarily, only the highest 5-CU concentration (4 mg/ml) induced an important reduction in cell viability (75% of cell death).

Combined with the above Hoescht test results, the neutral red results can be interpreted as differential cell death mechanisms involved by 5-CU treatment as compared to 5-FU treatment. Indeed, 5-CU seems to induce apoptotic cell death with an IC₅₀ of 2.5mg/ml after 72 hours of exposure. 5-FU rather induces apopto-necrotic cell death with an IC₅₀ of 7 µg/ml after 72 hours of exposure. 5-CU is therefore much less toxic on non-proliferative cells than is 5-FU.

### Example 2: Effect of 5-CU on rabbit fibroblast viability and proliferation

Before performing *in vivo* experiments in rabbits, the effect of a 24 hour-incubation of 5-CU on rabbit Tenon's capsule fibroblasts viability has been investigated and compared to the effects of an incubation with 5-FU. Further, the antimitotic effect of 5-CU has been evaluated and compared to that of 5-FU for preventing glaucoma surgery failure, and ability to prevent cell proliferation.

### Isolation and Culture of rabbit Tenon fibroblasts

A few pieces of Tenon's capsule were isolated from rabbit eyes. Tenon's capsule fibroblasts were dissociated and cultured in Dulbecco's MOD Eagle medium (Gibco BRL, Cergy Pontoise, France), supplemented with 10% FBS (Dominique Dutscher, Brumath, France), 1 % penicillin-streptomycin (Eurobio, Les Ulis, France) and 1% glutamine (Eurobio, Les Ulis, France), at 37°C, in a humidified incubator saturated with 5% CO₂ and 95% air. Cells were seeded at a density of 2 x 10⁴ cells / well until reaching subconfluency and were then treated with 5-chlorouracil (5-CU) or with 5-fluorouracil (5-FU) at different concentrations (0, 0.5, 5, 50, 500, 1'000 or 5'000 µg/ml) for 24 hours. Cell counting and MTT were used to determine effects on cell proliferation and survival as described below.

### Colorimetric MTT (tetrazolium) assay

MTT (Sigma) (1mg/ml in PBS) was added to the wells and plates were incubated at 37°C for 1 hour. Isopropanol was then added and mixed thoroughly to dissolve the dark blue crystals and the plates were rapidly read on a BIO-RAD Model 450 Microplate reader, using a test wavelength of 570 nm and a reference wavelength of 630 nm.

### Trypan blue exclusion test

In order to determine the effect of 5-CU and 5-FU on the proliferation of rabbit fibroblasts, cells were seeded on 24-well plates and treated after 24 hours with different concentrations of 5-FU and 5-CU (0, 1, 10, 20 or 100 µg/ml), diluted in culture medium. After 24 hours, control and treated cells were washed with PBS, trypsinized, stained with trypan blue 0.1-0.2% (Eurobio, solution at 0.4%), and the number of viable (uncolored) and dead (colored) cells was scored. The ratio of the number of uncolored/all cells gives the percentage of viable cells.

### Statistical Analysis.

Results were expressed as mean ± standard error of the mean (SEM). Statistical analyses were performed using the Mann-Whitney non-parametric test.

### Rabbit fibroblast viability:

The toxicity of 5-CU on sub-confluent fibroblasts is reduced compared to that of 5-FU since the highest 5-CU concentration (5'000 µg/ml) induced only 33% of cell death. 5-FU induces significant cell death at 5 µg/ml (39.1%) and 50 µg/ml (51%). Thus, 5-CU is 1'000 times less toxic to non-proliferating cells than 5-FU (Figure 1).

### Inhibition of rabbit fibroblasts proliferation:

Cell counting after a 24 hours exposure on proliferating cells shows that 5-FU and 5-CU induce dose-dependant inhibition of cell proliferation. This inhibition is significant with a concentration of the agents of 10 µg/ml or more. At a concentration of 10 µg/ml, 5-FU and 5-CU inhibit respectively 28 % (P=0.007) and 23 % (P=0.03) of cell proliferation. With 20 µg/ml, the inhibitions are respectively 40% (P=0.0018) and 23 % (P=0.03); and with 100 µg/ml, 43% (P=0.0014) and 25 % (P=0.009) (Figure 2). This experiment demonstrates that 5-CU is efficient to significantly prevent cell proliferation at a concentration as low as 10µg/ml. However, from 10 to 100 µg/ml, 5-CU is less antimitotic than 5-FU.

This further indicates that 5-CU concentrations in a range of or about 10 to or about 1'000 µg/ml at the site of injection are beneficial for the treatment of glaucoma by resulting in an efficient inhibition of Tenon's fibroblasts proliferation, while achieving reduced associated collateral toxicity and reduced local inflammatory reaction as compared to known substances used for the treatment of glaucoma post-filtering surgery.

### Example 3: Slow release formulation of 5-CU in poly(ortho esters)

A 5-CU pharmaceutical formulation according to the invention was prepared as follows:

### Materials

Diketene acetal 3,9-diethylidene-2, 4, 8, 10 - tetraoxaspiro [5,5] undecane (DETOSU) was furnished by Innovation and Chimie Fine, Manosque, France. Triethylamine (TEA), p-toluenesulfonic acid (p-TSA) and 1-decanol were purchased from Fluka (Chemie AG, Buchs, Switzerland). 1,10-decanediol was purchased from Aldrich (Chemie, Steinheim, Germany) and DL-lactide from Purac. 5-chlorouracil (5-CU) was purchased from Sigma (Chemie AG, Buchs, Switzerland). Solvents such as tetrahydrofuran (THF) and methanol were of analytical grade.

### Polymer synthesis

1,10-decanediol-lactate is synthesized by ring opening of lactide as described previously (Schwarch-Abdellaoui, 1999, J. Biomater. Sci. Polym. Edn. 10, 375-389). 7,205 g of DL-lactide (50 × 10⁻³) (Aldrich) and 8.713 g of 1,10-decanediol (Aldrich) (50 × 10⁻³) were introduced into a round-bottom flask sealed with a rubber septum under argon atmosphere. The mixture was vigorously stirred for 3 days at 160 °C. Viscous diol-lactate was used in the polymerization without further purification.
The synthesis of auto-catalyzed POE₇₀LA₃₀ (POE IV) containing 30 mol % lactic acid units in the polymer backbone has been previously described (Schwarch-Abdellaoui, 2002, Int. J. Polymer Analysis and Characterization, 7, 145-161). Briefly, POE IV was synthesized under anhydrous conditions (THF) by the acid catalyzed condensation of DETOSU (50 mmol) with 1,10-decanediol (20 mmol), 1,10-decanediol-lactate (15 mmol) and *n*-decanol (15 mmol) used as chain stopper as described previously (*Schwarch-Abdellaoui, 2002, above* and WO 01/85139). Low molecular weight oligomers, unreacted monomers and catalyst were removed by dissolution-precipitation using THF and methanol as solvent and non-solvent. The precipitate was dried under vacuum at 40°C for 48 h. Average molecular weights were determined by size exclusion chromatography (SEC) using polystyrene standards for the calibration curve.

### Preparation of the formulations

Formulations were prepared under a laminar airflow hood. The added drug, 5-CU or 5-FU (Sigma, Buchs, Switzerland) had been gamma sterilized at 2.0 MRad and homogeneously dispersed in the aseptically prepared semi-solid polymer under aseptic conditions at a concentration of 1% wt/wt. The viscous mixture was conditioned in a 1.0 ml syringe, each sample being 200µl (240 mg).
In a preliminary investigation, the *in vitro* rate of release of 5-CU was determined according to the procedure described by Merkli et al., 1998, J. Control. Release, 55, 213-8, it was evaluated at 8 micrograms per day over a period of up to 40 days. 5-FU *in vitro* rate of release from POE IV has also previously been described by Heller et al, 2005, Adv. Drug Delivery Rev., 57(14)2053-62*.* It was evaluated at 100 µg per day, under the same conditions (*Heller et al., 2005, above*).

It has been found that 1% 5-CU is released from the above POE IV formulation according to the invention with a daily release rate of approximately 8 µg/day, for up to forty days. The release is slow, controlled and continuous over the period required following a surgical intervention, in order to have an inhibitory effect on all fibroblasts in the appropriate phase.

### Example 4: Effect of by 5-CU on intraocular pressure post-trabeculectomy in rabbits

In this experiment, the efficiency of a slow release of 5-CU (5-CU was formulated according to a pharmaceutical formulation according to the invention) in maintaining the postoperative IOP below its preoperative value, as compared to 5-FU, was evaluated.

Pigmented Fauve de Bourgogne female rabbits weighing from 2 to 3 kg, 10 to 12 weeks of age, were used in this study (Elevage des Pins, Epeigne sur Deme, France). All experiments were conducted in accordance with the ARVO Statement for the Use of Animals in Ophthalmic and Vision Research. Rabbits received general anesthesia (50 mg/kg ketamine and 15 mg/kg xylasine intramuscular) and local anesthesia was obtained with oxybuprocaïne 0.4 %. At the end of the experiments, rabbits were sacrificed by intracardiac injection of a lethal dose of pentobarbital.
24 rabbits underwent trabeculectomy and were divided up in four groups (n=6): **(i)** trabeculectomy alone (Group 1); **(ii)** per-operative sub-conjunctival injection of 200 µl of POE IV alone (Group 2); **(iii)** per-operative sub-conjunctival injection of 200 µl of 5-fluorouracil POE IV formulation prepared according to Example 3 (Group 3); **(iiii)** per-operative subconjunctival injection of 200 µl of 5-chlorouracil POE IV formulation prepared according to Example 3 (Group 4). Intraocular pressure (IOP) and filtration bleb persistence were monitored during 34 days.
Another group of four rabbits underwent trabeculectomy on the right eye. Two of the rabbits did not receive any adjunctive treatment whilst two of them received 200 µl of the 5-CU formulation according to Example 3. Rabbits in the control group were sacrificed acutely (n=1) and at 11 months post-surgery, whilst the treated rabbits were sacrificed at 11 months. Fresh eyes were examined *ex vivo* using high frequency ultrasonography as described below.

### Glaucoma Filtering Surgery Procedure

Trabeculectromy was performed as previously described (Einmahl et al., 2001, Invest. Ophthalmol. Vis. Sci., 42, 695-700). Briefly, a half-thickness, limbus-based, 4x4 mm scleral flap was extended just anterior to the limbus, and was flipped-over. A 3 mm long limbal incision was made with a 45° blade that entered the anterior chamber. A piece of tissue containing inner sclera, trabeculum and peripheral cornea, measuring approximately 3x1 mm, was excised. A peripheral iridectomy was performed. The scleral flap was sutured with two 10-0 nylon sutures. The conjunctiva was repositioned with a continuous suture of 8-0 Vicryl (Ethicon, Ethnor, Neuilly France)

### Subconjunctival Injections

Just before closing the last conjunctival suture, a 0.9 mm diameter metallic needle (20 Gauge) was inserted in the sub-conjunctival space. 200 µl of POE IV (Group 2), or POE IV/1 % wt/wt 5-FU (Group 3) or of the 5-CU formulation according to Example 3, i.e. POE IV/1 % wt/wt 5-CU (Group 4) were injected at the site of the trabeculectomy, as previously described (*Einmahl et al., 2001, above*).

### Clinical Follow-Up

In the four groups, slit-lamp observations were performed at 5, 8, 15, 22, 27, 34 days after surgery, in order to evaluate the filtering bleb persistence. At 1, 5, 15, 34 days, IOP was measured using Goldmann aplanation tonometry, and was compared with the pre-operative IOP. In the POE IV/5-CU and the control groups, the IOP evolution was followed for a period of 5 months.
Statistical analysis was performed to compare experimental data with data from control eyes, using a non-parametric Mann-Whitney test. IOP and time to bleb failure were analyzed. P<0.05 was considered significant.

### Ex vivo ultrasonographic evaluation of the filtration site

During ultrasonography examination, the eyeball was maintained immobilized in an appropriate position by two surgical sewing threads placed at the limbus, opposite to each other. The eyeball was immersed in a saline (0.9%) filled tank, and positioned on an adjustable holder. The eye was oriented such that the trabeculectomy site was facing the transducer. The eyes are examined using a custom-built three-dimensional ultrasound backscatter microscope (Lheureux et al., 2000, Ultrasonics, 39, 391-5) operating with a single element polymer focused transducer (Toray Techno Co., Shira-ken, Japan). The transducer (80 MHz nominal frequency, 7.5-mm focal length, 3-mm aperture) was excited by a pulser-receiver (Parametrics, Inc., Waltham, USA, model 5900PR) to generate a wide bandwidth pulse at focus. The amplitude spectrum of the ultrasound signal reflected by a perfect steel plane placed at the focus was centered at 50 MHz. The -6 dB axial and lateral resolutions were 20 and 65 µm, respectively. The transducer scanning was carried out by a micrometric stepping motors allowing lateral and vertical (ultrasound beam axis) translation (Microcontrôle Newport, Evry, France; model TL78) providing a 0.1-µm positional resolution. The radiofrequency (RF) signal back-scattered by the tissue was acquired, under fixed electronic settings, at 400 MHz sample rate by a 8-bits digital oscilloscope (LeCroy Co., NY, USA, model 9450A), then transferred to a personal computer for B-scan image reconstruction and visualization, and data processing. Three-dimensional information from the operated site was obtained by scanning the transducer along the anterior-to-posterior direction.

### Intraocular pressure

Following trabeculectomy, IOP is supposed to be and stay below the preoperative value (POV) (8.2 mmHg). In Group 1, that underwent trabeculectomy alone (1), the mean IOP decreased immediately after surgery, stayed approximately steady until day 15, but finally returned to its pre-operative level by day 28. In eyes which received POE IV alone (Group 2, (2)), IOP also returned to pre-operative value, but with some advance, at day 8. At the end of this experiment, i.e. at day 34, mean IOP was still below its preoperative value in both treated groups (Groups 3 and 4): (3) = POE IV + 5-FU formulation prepared according to Example 3; (4) = POE IV + 5-CU formulation prepared according to Example 3. At this time point, mean IOP was 83% of the baseline value in the POE IV/5-CU treated group (Group 4), and 40% in the POE IV/5-FU treated group (Group 3).
A long-term follow-up of IOP (Figure 3) shows that, 98 days after surgery, IOP is still decreased in the Group 4 (POE IV/5-CU) (4) when compared to the controls ((1) and (2)), and still inferior to the preoperative value (POV). The mean long term IOP (Figure 4), all time points being added up together, is extremely significantly (P<0.0001) decreased in the POE IV/5-CU treated group when compared to both control groups.

Slit lamp examination, 4 weeks after filtering surgery and bleb survival curves (Figures 5A and 5B) shows that no filtrating blebs could be detected in the Group 1 (trabeculectomy alone) (Figure 5A, top), whereas a well-shaped bleb could be observed in the Group 4 (trabeculectomy + POE IV/ 1% 5-CU) (Figure 5A, bottom). Fifty percent of blebs had collapsed by day 17 in Group 1 (control eyes that underwent trabeculectomy alone), and 13.3% were still filtrating by 34 days (Figure 5B(1)). In Group 2 (eyes that received POE IV alone), fifty percent of blebs had collapsed by 21 days, and 81.8% by 34 days; only 18.2% of blebs were still functioning in this Group (2) at day 34(Figure 5B). Group 3, which received POE IV formulation containing 5-FU, showed delayed persistence of the blebs, compared with Groups 1 and 2: 80% were still filtrating at day 34. All blebs in Group 4 (POE IV + 5-CU) survived until postoperative day 22, and 80% survived after 1 month (Figure 5B).

B-scan image of a control trabeculectomized eye (Figure 6 after trabeculectomy, removing of scleral and iridal tissues shows clearly two distinct hypoechoic areas (Figure 6). At 11 months post-surgery, the operated site of the control eye sclera can't be spotted anymore (Figure 6). At this time point, the trabeculectomy site is still visualized by a hypoechoic area in the POE IV/5-CU injected eye (Figure 6).

The *in vivo* effect of this 5-CU formulation according to the invention is impressive with consistently lower IOP levels in eyes that have undergone trabeculectomy without 5-CU formulation injection. This reduced IOP is maintained for up to 5 months following filtration surgery. Previous studies on animal models using other antiproliferative agents in single dose application have not yielded such promising long-term results. Single application of antiproliferative agents and sub-conjunctival injections are the only method currently available. Earlier studies on 5-FU in tissue culture showed continuing inhibition of proliferation of fibroblasts after discontinuation of treatment either if exposure was sufficiently long or concentrations sufficiently high. However, toxicity to proliferating corneal epithelial cells is an important limiting factor in the application of high concentrations or over a prolonged period of 5-FU. Also, the effects in tissue culture are not necessarily applicable to the clinical setting where the inflammatory response will lead to new fibroblasts in different stages of the cell cycle entering the operated site in the first two weeks following surgery. Sub-conjunctival injections of 5-FU maintain ID₅₀ levels up to 24 hours at the injection site and 180° from it, following injection, with the disadvantage that corneal levels are also high (20 µg/g at 1 hour, 5 µg/g at 24 hours). With frequent topical administration, therapeutic levels can also be maintained, unfortunately with toxic inhibition of corneal epithelial proliferation.

Altogether, these results show that 5-CU is an anti-fibroblastic agent developed for the first time for use in glaucoma filtration surgery. 5-CU is also less toxic to normal epithelial cells. It can be combined with a slow release biodegradable delivery system such as poly(ortho esters) and be administered following trabeculectomy to provide prolonged anti-fibroblastic activity and excellent long-term control of IOP with less corneal toxicity than previously used agents.

Moreover, these results show that when 5-CU is administered, *in situ* cell death is more oriented toward apoptosis, when compared to 5-FU administration. This, also, is a striking advantage, because apoptosis is less responsible for inflammation than necrosis; so apoptosis is less likely to promote excessive wound healing. Formulations of 5-CU according to the invention allow to dissociate the therapeutic inhibition of sub-conjunctival proliferation from toxic inhibition of normal conjunctival and corneal cells by delivering the therapeutic agent in a steady and controlled fashion at the desired location (cellular toxicity must be limited to proliferating cells and should not induce any collateral inflammatory response).

Further, the biodegradable formulations according to the invention have excellent biocompatibility profile, which allows a continuous release of 5-CU in the sub-conjunctival space, over the early postoperative period in glaucoma filtering surgery.

## Claims

1. Use of 5-chlorouracil for the preparation of a pharmaceutical composition for the treatment of glaucoma.

2. A use according to claim 1 wherein the composition is an ocular formulation suitable for ocular injection.

3. A use according to claims 1 or 2 wherein the composition is an ocular formulation suitable for sub-conjunctival ocular injection.

4. A pharmaceutical composition comprising a therapeutically effective amount of 5-chlorouracil, poly(ortho esters) and a pharmaceutically acceptable carrier or excipient.

5. A pharmaceutical composition according to claim 4 wherein the poly(ortho esters) are selected from poly(ortho esters) III and poly(ortho esters) IV.

6. A pharmaceutical composition according to claims 4 or 5 wherein the pharmaceutical composition comprises a weight amount of 5-chlorouracil selected from about 1, 2, 3, 4, 5, 6, 7, 8, 9 and 10% (wt/wt).

7. A pharmaceutical composition according to any one of claims 4 to 6 wherein the pharmaceutical composition comprises a weight amount of 5-chlorouracil of about 1% (wt/wt).

8. A pharmaceutical composition according to any one of claims 4 to 7 wherein the poly(ortho esters) are poly(ortho esters) is a low molecular weight autocatalyzed POEₓLA_{y} wherein x ranges from about 70 to about 90 and y ranges from about 30 to about 10.

9. A pharmaceutical composition according to any one of claims 4 to 8 further comprising a co-agent useful in the treatment of glaucoma and/or glaucoma post-operative filtering surgery.

10. A use according to any one of claims 1 to 3, wherein the composition is a pharmaceutical formulation according to any one of claims 4 to 9.

11. An administration kit comprising: (a) a container containing 5-chlorouracil or a pharmaceutical formulation according to any one of claims 4 to 9; and (b) a container containing a pharmaceutically acceptable medium solution.

12. A biodegradable implant coated with for placement in an eye coated with a therapeutically effective amount of 5-chlorouracil.

13. A biodegradable implant according to claim 12 wherein the implant is coated with a pharmaceutical formulation according to any one of claims 4 to 9.

14. An implant kit comprising: (a) an implant according to claims 12 or 13; and (b) a delivery apparatus for the said implant.
